# EUROPÄISCHE PATENTSCHRIFT

(11) **EP 2 175 892 B1**
(45) Veröffentlichungstag und Bekanntmachung des Hinweises auf die Patenterteilung: **26.09.2012**
(21) Anmeldenummer: 08802978.0
(22) Anmeldetag: 08.08.2008
(51) Int. Cl.: A61L 2/10, A61L 2/14, A61L 2/20, A61L 9/015, A61L 9/20, A61L 9/22, D06L 1/00, D06L 3/00, D06L 3/04, D06M 10/00, D06M 10/02, D06M 10/06, D06M 16/00

(54) **VERFAHREN ZUR TEXTILREINIGUNG UND DESINFEKTION MITTELS PLASMA UND PLASMASCHLEUSE**
PROCESS FOR TEXTILE CLEANING AND DISINFECTION BY MEANS OF PLASMA AND PLASMA LOCK
PROCÉDÉ DE NETTOYAGE ET DÉSINFECTION DE TEXTILES PAR PLASMA ET SAS À PLASMA

(30) Priorität: 10.08.2007 DE 102007037984
(43) Veröffentlichungstag der Anmeldung: 21.04.2010
(73) Patentinhaber: Leibniz-Institut für Plasmaforschung und Technologie e.V., 17489 Greifswald (DE)
(72) Erfinder: MÜLLER, Siegfried, 17493 Greifswald (DE); ZAHN, Rolf-Jürgen, 17493 Greifswald (DE); REICH, Wolfgang, 17489 Greifswald (DE); WELTMANN, Klaus-Dieter, 18609 Ostseebad Binz (DE); ANKLAM, Kirsten, 18519 Bremerhagen (DE); NEUDECK, Diana, 15432 Stahnsdorf (DE); KRÜGER, Tila, 17506 Gützkow (DE); MLECZKO, Norman, 24539 Neumünster (DE); KOBURGER, Torsten, 17489 Greifswald (DE); HARFENSTEIN, Ivonne, OT Lebbin, 17091 Gross Teetzleben (DE)
(74) Vertreter: Garrels, Sabine
(86) Internationale Anmeldenummer: PCT/EP2008/060453
(87) Internationale Veröffentlichungsnummer: WO 2009/021919

(56) Entgegenhaltungen:
- DE-T2- 60 307 062
- US-A1- 2003 030 374
- US-A1- 2006 096 331
- US-B1- 6 451 252

## Beschreibung

Die Erfindung betrifft ein Verfahren zur Textilreinigung und Desinfektion mittels Plasma und stellt einen neuen Weg zur wasserfreien Waschmaschine dar. Die Erfindung betrifft weiterhin eine Schleuse oder Raum zur Inaktivierung von Viren, Bakterien und Sporen als auch zur Reinigung von Luft im üblichen Sinne wie von Staub, Pollen, Gerüchen oder dergleichen.

### Stand der Technik

Es bestehen verschiedene Gefährdungen z.B. durch belastete Luft. Neben gasförmigen Komponenten und Aerosolen antropogenen Ursprungs, zählen dazu insbesondere biologische Komponenten wie Viren, Sporen, Bakterien oder Pollen. Viren und Bakterien können aus verseuchten Gebieten durch Lebewesen, Fahrzeuge oder Stückgüter verschleppt werden. In jüngster Zeit sind vor allem virologische Gefährdungen wie H5N1 (Vogelgrippe), Influenza, oder SARS ins Blickfeld gerückt, da insbesondere Pandemien mit zahlreichen Opfern zu befürchten sind. Im Normalfall ist eine ausreichend hohe Konzentration des infektiösen Materials für eine Ansteckung erforderlich.

Neben Raumluft oder Umgebungsluft bilden Abgase oder Abluft häufig erhebliche Belastungen für die Gesundheit und Umwelt. In vielen Fällen sind die Abgase oder die Abluft wiederum mit Aerosolen beladen. Diese können zusätzlich Träger von belästigenden Gerüchen oder VOC's (volatile organic compounds) sein. Unter VOC werden schädliche Kohlenwasserstoffverbindungen verstanden.

Aerosole ist der Sammelbegriff für feste und flüssige Partikel. Insbesondere kleinere Aerosole im Bereich weniger nm sind besonders gesundheitsschädlich. Diese oft als Nanopartikel bezeichneten Verunreinigungen können ungehindert in die menschliche Lunge eindringen oder auch direkt über die Haut aufgenommen werden. Bekanntestes Beispiel solcher Nanopartikel ist der Ruß in Dieselabgasen. In Raumluft sind ferner oft schädliche Stoffe wie Partikel von Zigarettenrauch, Toner von Photokopierern oder Papierstaub von Kopierpapier enthalten.

Zur Reduzierung der beschriebenen Belastungen bei Abgasen oder Abluft werden verschiedene Wege beschritten. Übliche Mittel wie mechanische Abscheider, mechanische Filter, Wäscher, oder die Verbrennung sind nur begrenzt einsetzbar, zu aufwendig oder haben einen zu hohen Energieaufwand.

Speziell für die Aerosolabscheidung, insbesondere Beseitigung makroskopischer Staubteilchen aus industriellen Abgasen, sind elektrostatische Filter oder auch Elektrofilter zur Reinigung bekannt. Dieses auch als elektrische Gasreinigung bezeichnete Verfahren enthält als einen üblichen Verfahrensschritt die elektrische Aufladung der Staubteilchen mit einer Koronaentladung. Eine typische Geometrie besteht aus einem dünnen Draht, der von einem weit entfernten Zylinder umgeben ist. Je nach Polarität der Drahtelektrode unterscheidet man die negative oder positive Korona. Bei der negativen Korona führt Elektronenanlagerung insbesondere zur Erzeugung negativer Ionen, die sich gegebenenfalls an Aerosole anlagern. Dieser Prozess wird manchmal auch als Ionenwind bezeichnet.

Elektrofilter besitzen sogenannte Abscheideelektroden und Sprühelektroden mit entsprechenden Hochspannungsfeldern dazwischen. Die auf der Abscheideelektrode gesammelten Staubteilchen werden mechanisch entfernt, was in bestimmten Anwendungen nachteilig ist. Auch die für Elektrofilter erforderlichen hohen Spannungen von mehreren 10 kV sind nachteilig.

In DE 34 20 973 A1 wird eine Kombination mit Filtermatten vorgeschlagen, auf denen der abgeschiedene Staub gesammelt werden kann.

In DE 102 45 902 A1 ist vorgesehen, dass die Abscheideelektrode einen Raum umfasst, in den die Partikel eintreten können und in dem kein Potentialunterschied herrscht.

In DE 30 04 474 C2 wird der unterhalb der Durchbruchspannung liegenden Koronagrundspannung eine Pulsspannung mit Pulsbreiten im ns- bis ms-Bereich überlagert.

Nach DE 43 39 611 A1 soll eine Staubaufladung im ganzen Volumen, ein homogenes Abscheidefeld, und Verhinderung von elektrischen Durchschlägen dadurch erreicht werden, dass eine Segmentierung in Strömungsrichtung, bei der sich dielektrisch behinderte Entladungsstrecken zur Aufladung der Staubpartikel mit "normalen" Abscheidestrecken mit hohen homogenen elektrischen Feldern zwischen metallischen Elektroden abwechseln. Die dielektrisch behinderte Entladung wird zwischen einer Dielektrikumselektrode und der Abscheideelektrode betrieben, also über den ganzen Querschnitt des Gasraumes. Damit sind in dieser Anordnung, ähnlich wie bei einer Koronaentladung, bei einem größeren Abgaskanal zur Erzielung eines kleinen Strömungswiderstandes sehr hohe Spannungen (in diesem Fall Wechselspannungen) erforderlich. Für den Koronateil bleiben die üblichen Probleme bestehen.

Ganz allgemein ist auch bekannt, dass mit Plasmen verschiedenste chemische Reaktionen initiiert werden können, die vor allem über sehr reaktive Spezies, sogenannte Radikale, ablaufen. Dies hat man für verschiedene Anwendungen zur Behandlung von Abgasen oder auch für plasmachemische Reaktoren untersucht und genutzt. Es sind dabei Lösungen vorgeschlagen, die eine dielektrisch behinderte Entladung zur Generierung geeigneter Plasmen ausnutzen. Dielektrisch behinderte Entladungen (nachfolgend auch DBE) sind dadurch gekennzeichnet, dass mindestens eine der leitfähigen Elektroden mit einem Dielektrikum versehen ist, welche dann eine isolierte Elektrode bildet, oder dass ein Dielektrikum zwischen den leitfähigen Elektroden angeordnet ist. Die Form solcher Anordnungen kann vielgestaltig sein. Abhängig von dieser Form und den übrigen Parametern werden dann oft spezifische Eigenschaften der DBE erreicht. Manchmal werden spezielle Bezeichnungen nach solchen Formen oder auch nach dem Zwecke der Anwendung (z.B. Ozonisator) benutzt.

Generell kann die DBE mit sinus- oder rechteckförmigen Wechselspannungen im Bereich von einigen Hz bis zu mehreren 100 kHz betrieben werden. Als Entladungsform sind verschiedene Ausbildungen der DBE bekannt. Häufig werden bei großflächigen Elektroden zumeist statistisch verteilt zahlreiche kleine bis zu wenigen zehntel mm dicke Entladungsfäden, auch Filamente genannt, ausgebildet. Die Filamente bilden im Übergangsbereich zu den isolierten Elektroden Aufweitungen aus, die häufig in Oberflächengleitentladungen mit zahlreichen weiteren dünnen Entladungskanälen übergehen.

Solche Gleitentladungsphänomene können in speziellen Anordnungen, sogenannten koplanaren oder Oberflächenentladungsanordnungen, dominant sein. Bekannt ist auch, dass insbesondere in Gasfüllungen mit Edelgasen bzw. ihren Gemischen homogene, nicht filamentierte Entladungsstrukturen ausgebildet werden können. Daneben sind verschiedene Kombinationen und Übergangsformen von Entladungsausbildungen möglich.

Bei nach dem Stand der Technik aufgebauten Reaktoren können insbesondere Aerosole schlecht zurückgehalten und im Plasma zersetzt werden.

Für eine effektive Behandlung von Rußpartikel ist nach DE 197 17 890 C1 vorgesehen, Ruß auf einem porösen Filterelement zu sammeln und dem Plasma einer DBE auszusetzen. Ein ähnliches Prinzip ist in DE 100 57 862 C1 angegeben. Bei diesem Prinzip bleibt das Problem der Ascheablagerung und Verstopfung bestehen.

Aus der WO 2005/028081 bzw. DE 103 44 489 A1 ist bekannt, dass eine zusammenhängende, in alle Raumrichtungen strukturierte Elektrode benutzt wird, auf deren Erhebungen ein Isolierstoff liegt. Dieser Isolierstoff bildet für die strukturierte Elektrode eine begrenzende Fläche. Auf der anderen Seite des Isolierstoffes ist eine weitere Elektrode angebracht. Die strukturierten Elektrode besteht aus einem Drahtgeflecht, zusammenhängenden oder aneinander liegenden Körpern bzw. Gebilden aus einem elektrisch leitfähigen Material. Die strukturierte Elektrode fungiert dabei gleichzeitig als Abstandshalter für weitere Ebenen und als Filterelement. Die Entladung, die eine spezielle Form einer DBE darstellt, bildet sich in den Zwischenräumen der Elektrodenstruktur und auf der Oberfläche des Isolierstoffes aus.

Für einige Anwendungen bewirkt die Struktur einen zu hohen Gegendruck. Außerdem sind die Reaktionen im Plasma oft sehr komplex , da alle im Plasma erzeugten Spezies an den Reaktionen beteiligt sein können.

Bekannt ist ferner eine Anordnung nach DE 198 26 831 A1, wo der Plasmareaktor aus einer rohrförmigen Elektrode besteht, die auf ihrer Innenfläche mit einer dielektrischen Barriere beschichtet ist. Als Gegenelektrode ist im Kontakt mit der Barriere eine dem Gasraum zugewandte leitende Elektrode, beispielsweise aus einem Drahtgitter, angebracht. Bei entsprechenden Spannungen kommt es dann im Spaltbereich der Drahtgitterelektrode und der dielektrischen Barriere zur Ausbildung von Gasentladungen. Eine Vermischung des einströmenden Gases und des Plasmabereiches erfolgt durch Wirbeleffekte im unmittelbaren Bereich der Drahtgitterelektrode.

In der DE 196 16 206 A1 wird eine Gasentladung vornehmlich an der Oberfläche eines Dielektrikums erzeugt, wobei die dem Gas zugewandte Elektrode unmittelbaren Kontakt mit dem Dielektrikum besitzt und aus dünnem Stabmaterial mit abgerundetem Querschnitt besteht. Katalytisch wirkende Materialien befinden sich in einigen mm Abstand zu dieser dem Gas zugewandten Elektrode und befinden sich auf gleichem Potential.

Bei diesen Anordnungen ist für viele Anwendungen nachteilig, dass der Austausch zwischen Plasma und übrigem Gas eingeschränkt ist, und damit auch für plasmainduzierte Reaktionen im Gas. Es können ferner keine Aerosole oder Rußpartikel abgeschieden werden, da keine Filterung oder Abscheidung vorhanden ist. In der Folge werden damit im Plasma solcher Anordnungen Aerosole/Rußpartikel auch nicht erfolgreich zersetzt.

Von der Anmelderin ist ferner eine Anordnung und Verfahren bekannt, wo ein Plasma mit einer dielektrisch behinderten Entladungsanordnung in einem großflächigen Bereich erzeugt wird, wobei zur Seite des Gasraumes für das einströmende Gas hin eine offene Struktur der DBE vorhanden ist. Aus dem Plasma der DBE werden Ionen in einem elektrischen Feld verfahrensgemäß extrahiert und beschleunigt, so dass ein Ionenwind entsteht und den Raum für das einströmende Abgas bzw. die Abluft erfasst. In einer einfachen Ausgestaltung wird das elektrische Feld zur Ionenextraktion durch negative Pulsspannungen des das Plasma erzeugenden Teiles einer dielektrisch behinderten Entladung gebildet. Die dieser DBE-Anordnung gegenüberliegende Seite der Vorrichtung befindet sich auf Erdpotential. Negative Ionen werden zum Erdpotential hin beschleunigt, so dass im Gas enthaltene Aerosole aufgeladen und abgeschieden werden können. In einer anderen Ausgestaltung ist dabei, neben der Abscheidung von Aerosolen, auch deren Zersetzung im Plasma vorgesehen. Dazu werden sich gegenüberstehende dielektrisch behinderten Entladungsanordnungen ausgebildet. Die sich zum Gasraum für das einströmende Gas gegenüberliegenden Seiten der DBE-Anordnungen werden dabei mit positiven bzw. negativen Pulsspannungen beaufschlagt. Auf diese Weise driften Ladungsträger sowie aufgeladene Aerosole je nach Vorzeichen ihrer Ladung zu der jeweils entgegengesetztes Potential führenden Elektrode der DBE hin. Dort werden die Aerosole abgeschieden und im Plasma der jeweiligen DBE zersetzt.

US-B1-6451252 befasst sich mit der Beseitigung von Geruchskomponenten, indem ein Gas-Aufbereitungs-Weg zwischen Gas-Einlass, und Gas-Auslass für die Behandlung vorgesehen ist. In einem Mischer wird das zu behandelnde Gas mit Ozon aus einem OzonGenerator vermischt und dann einem nicht-thermischen Plasmareaktor zugeführt. Die gesamte Schrift bezieht sich auf die Behandlung von "odorous gas", also von Geruchskomponenten im Gas, und nicht mit der Entkeimung von Gas, was neben dem Geruch auch andere Komponenten meint wie Bakterien usw.

In DE 60307062 T2 wird ein Verfahren zur Plasmareinigung der Oberfläche eines mit einer organischen Substanz beschichteten Materials, insbesondere von Metallblechen, offenbart. Hierzu wird das Material in einem Behandlungsbehälter unter einem Druck zwischen 10 mbar und 1 bar und der Zufuhr von mindestens 90 Vol.-% Sauerstoff gebracht. Die organische Substanz soll so unter Einwirkung der durch ein Plasma erzeugten freien Radikale O* zersetzt werden. Es wird in einer Ausführungsform die Redissoziation von Ozon zur Reinigung eines Substrates offenbart.

Solche Systeme erzeugen u.a. Ozon, so dass eine längere Anwesendheit von Lebewesen in solchen Räumen nicht möglich ist.

Bisherige Entkeimungs- und Luftreinigungssysteme haben verschiedene Einsatzgrenzen. Systeme auf der Basis von Plasmen arbeiten nach dem Umluft- oder Injektionsprinzip. Es wird dabei die Raumatmosphäre mit ihren schädlichen Bestandteilen im Wesentlichen im Reaktorteil durch reaktive Spezies behandelt. Im anderen Fall werden reaktive Bestandteile aus einer Plasmaeinheit mit in den Raum gegeben (wie etwa bei Entkeimung durch Ozon). Solche Systeme sind nur bedingt anwendbar und es sind so auch nur gasförmige oder gasgetragene Bestandteile behandelbar. Kritisch sind auch die Behandlungszeiten (und damit die Verweildauer) im Hinblick auf die Entkeimung zu sehen.

Eine sehr effektive Methode der Entkeimung/Luftreinigung bilden UV-Verfahren. Dabei ist aber eine direkte Entkeimung nur ohne Personenaufenthalt möglich ist. Auch Umluftbetrieb und indirekte Entkeimung sind möglich, aber mit ähnlichen Problemen wie zuvor.

Bei der Routinedesinfektion mit Desinfektionsmittel treten bei sinkender Temperatur erhebliche Wirksamkeitsverluste auf, so dass Durchfahrbecken oder Seuchenmatten in ihrer Wirksamkeit eingeschränkt werden. Ferner können diese Mittel nicht auf ungeschützte Personen gerichtet werden.

Die Reinigung und Desinfektion von Textilien ist mit dem Einsatz von Wasser und diversen Reinigungs- sowie Desinfektionsmittel verbunden. Das bedingt einen erheblichen Verbrauch unserer Wasserressourcen und führt zu vielfältigen Gewässerbelastungen. Danach sind aufwändige Abwasserreinigungsverfahren erforderlich.

Gegenstand und Verschmutzungsgrad sowie Art der Verschmutzung sind sehr unterschiedlich. So reicht die Palette der Anwendungen von einfacher Kleidung bis hin zu Reinraumtextilien, Medizintextilien oder gar Schutzausrüstungen. Die Art der Verschmutzung weist ein noch größeres Spektrum auf. Dieses reicht von einfacher Geruchsbeladung der Kleidung über die Verschmutzung mit Bioaerosolen (Keime, Sporen, Pollen) bis hin zu diversen anderen Verunreinigungen.

Die Behandlung von textilen Flächengebilden mittels Plasma ist aus den Patentveröffentlichungen DE 19634725 A1 und DE 3248590 A1 bekannt. Hier werden die Oberflächen von Textilien in der Art behandelt, dass bei einer später notwendigen Reinigung weniger Energie und Wasser benötigt werden. Eine vollständige Reinigung mittels Plasma erfolgt nicht.

Eine Möglichkeit der Oberflächenreinigung von Textilien mittels Plasma wird in JP 2005224757 A offenbart. Das Plasmareinigungsgerät soll Chemikalien und Waschmittel einsparen und besteht aus einem Bügeleisen mit einem Wassertank und Plasmaelektroden zur Erzeugung eines Plasmas unter Normaldruck. Der Wasserdampf wird durch das Plasma geführt, wobei Hydroxyl-Radikale entstehen, welche die Textilien reinigen.

Weiterhin gibt es Einrichtungen, mit welchen verunreinigte Luft mittels einer UV-Einheit und einer Ionisationseinheit entkeimt wird. Bekannte Vorrichtungen sind in DE 102005003923 A1 und in DE 102005035951 A1 beschrieben. Grundsätzlich sind bei dem Einsatz von UV-Strahlung und dem damit verbundenen Einsatz von Ozon folgende Reaktionsmechanismen bekannt. Molekularer Sauerstoff (O₂) wird in Sauerstoffradikale aufgespalten. Die so gebildeten Sauerstoffradikale reagieren ihrerseits wieder mit molekularem Sauerstoff unter Bildung von Ozon. Unter dem Einfluss von UV-Licht kann das so gebildete Ozon wieder in ein Sauerstoffradikal und molekularen Sauerstoff gespalten werden. Das Sauerstoffradikal steht nun zur Verfügung, um mit Wasser zu zwei Hydroxylradikalen oder mit molekularem Sauerstoff wieder zu Ozon zu reagieren.

Eine Anwendung für die Reinigung von Textilien ist hier nicht vorgesehen.

### Darstellung der Erfindung

Aufgabe der Erfindung ist es, ein Verfahren zur Reinigung und Desinfektion von Textilien zu entwickeln, bei dem der Einsatz von Wasser und diversen Reinigungs- sowie Desinfektionsmittel nicht erforderlich ist bzw. signifikant reduziert werden kann. Weiterhin ist es die Aufgabe, eine Schleuse zur Inaktivierung von Viren, Bakterien, Sporen als auch zur Reinigung von Luft im allgemeinen Sinne wie von Staub, Pollen, Gerüche etc. zu entwickeln. In der Schleuse sollen sich Lebewesen aufhalten können - ebenso sollen Stückgüter, Textilien usw. behandelt werden können. Dabei sollen luftgetragene Aerosole (Tröpfchen, Partikel, Staub) als auch der Kleidung oder dem Körper bzw. dem Produkt anhaftende Aerosole bzw. Keime behandelt werden. Das Grundprinzip soll auch allgemein auf Räume (z.B. Warteräume) oder Ställe sowie unter Reinraumbedingungen anwendbar sein.

Erfindungsgemäß erfolgt eine Reinigung von z.B. geruchsbeladener Kleidung sowie die Entkeimung von Textilien auf der Basis von Plasmaverfahren. Dabei werden folgende bekannte Verfahrensschritte eingesetzt:
a) Plasmagenerierung,
b) Ozonerzeugung und -aktivierung,
c) Entkeimung,
d) Oxidation und Zersetzung gasförmiger Komponenten,
e) Abscheidung von Keimen und Aerosolen und
f) ihre Zersetzung.

Erfindungsgemäß wird eine Plasmagenerierung benutzt, aus der Ionen durch Pulspannungen unterschiedlichen Vorzeichens extrahiert werden können. In einer Ausführungsform werden abwechselnd positive oder negative Ionen extrahiert.

Keime und Aerosole werden durch die extrahierten Ionen elektrisch aufgeladen und so durch ein elektrisches Feld einem Abscheidebereich oder einer Plasmaeinheit zur Zersetzung zugeführt.

Die Entkeimung erfolgt durch ein oder mehrere der folgenden Komponenten wie das erzeugte Ozon, den Aktivsauerstoff, die extrahierten Ionen und UV-Strahlung.

Eine Oxidation und Zersetzung gasförmiger Komponenten erfolgt durch aus dem Plasma extrahierte Ionen.

Es erfolgt eine zusätzliche Sauerstoffaktivierung durch eine UV-Lampe. Das UV-Licht hat eine Wellenlänge im Bereich von 220 bis 230 nm oder eine Wellenlänge im Bereich von 290 bis 315 nm oder eine Wellenlänge von 254 nm.

Zur Unterstützung der Wirksamkeit der Reinigung sind mechanische Mittel wie Luftzirkulation oder Klopfen sowie Wendevorrichtungen vorgesehen.

Gegenüber herkömmlichen Verfahren, wie z.B. konventionelles Waschen, chemische Reinigung/Desinfektion, chemisches Bleichen mit Chlor, liegen die Vorteile in der Einfachheit und im geringeren Aufwand. Wasser sowie Wasch- und Desinfektionsmittel und Chemikalien können bei der Reinigung verkeimter Textilien, anhaftender Gerüche sowie Verschmutzungen durch Staubaerosole, Sporen, Pollen in beträchtlichem Umfang eingespart werden.

Gelöst wird die Aufgabe durch Verfahren zur Ionenerzeugung und Ionenextraktion (Ionenwind - Transport der Ionen in den Behandlungsraum. Ferner der Aufladung und Abscheidung von Aerosolen/Keimen oder Anlagerung von Ionen und die Zersetzung/Oxidation von Keimen/Aerosolen über Ionen-Molekülreaktionen. Im Verfahren ist als weitere Komponente eine spezielle Kombination mit bekannten Entkeimungsverfahren vorgesehen.

Der wesentliche Nutzen liegt in der großen gesellschaftlichen Bedeutung hinsichtlich der Gesundheit. Das verfahren vermeidet große ökonomische Schäden in der Landwirtschaft. Ferner werden industrielle Prozesse ermöglicht oder verbessert.

### Kurze Beschreibung der Abbildungen

Die Erfindung wird anhand von Beispielen erläutert. Hierzu zeigen
- Figur 1: Sauerstoffaktivierung durch Bestrahlung von Ozon
- Figur 2: Textilentkeimung mittels aktiviertem Sauerstoff in verschiedenen Anordnungen
- Figur 3: Textilentkeimung - Frequenzabhängigkeit
- Figur 4: Reduktion der Geruchskonzentration in Textilien durch Plasmabehandlung
- Figur 5: Vorrichtung zum Reinigen leicht verschmutzter oder mit Gerüchen beladener Textilien
- Figur 6: schematische Funktionsweise der Düse
- Figur 7: Anordnung als Schleuse ausgebildet
- Figur 8: Schematische Darstellung der Plasmabehandlung im Stapelreaktor
- Figur 9: Diagramm Partikelkonzentration über die Zeit
- Figur 10: Diagramm des Vergleichs ohne (blau) und mit (rot) Plasma im Stapelreaktor(Stapelsystem mit 50 Gittern)

### Ausführung der Erfindung

Plasmagenerierung/Ozonerzeugung und -aktivierung,

Es werden energieeffiziente Verfahren und Anordnungen zur Plasmaerzeugung und nachfolgender Reaktionen (Oxidation) aufgezeigt. Insbesondere wird eine hohe Reaktivität erreicht, indem parallel mit einer Bestrahlung durch UV-Licht Ozon aktiviert wird, so dass daraus atomarer Sauerstoff entsteht. Dieser ist besonders reaktiv.

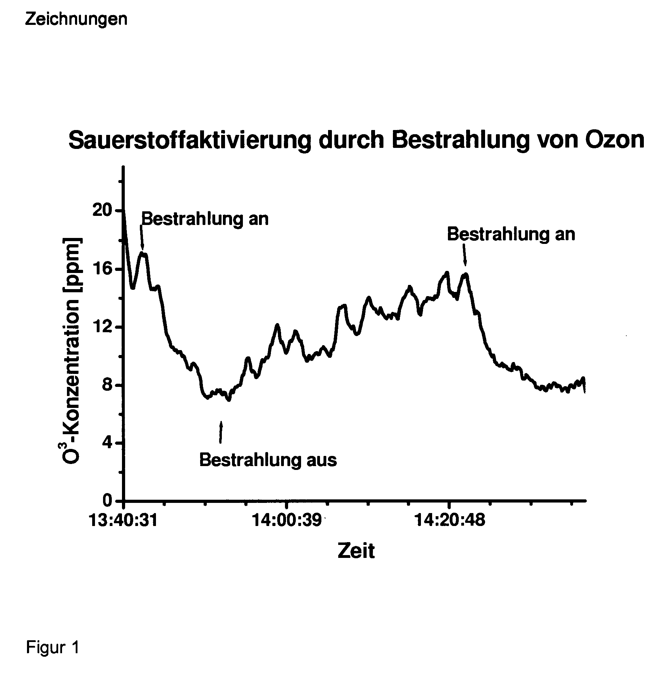

Figur 1 zeigt hierzu den rapiden Abfall der Ozon-Konzentration kurz nach Beginn der Bestrahlung durch UV-Licht. Wird das UV-Licht wieder ausgeschaltet, erhöht sich die Ozon-Konzentration wieder.

Entkeimung

Es wurden 2 wirksame Verfahren zur Entkeimung entwickelt. Bei den für Reinigungsverfahren besonders komplizierten Textilstrukturen gelang eine Keimreduktion von mit Bakterien E. coli versehener Prüftextilien um 5 Log.-Stufen, bei Aerosolen um 7 Log.-Stufen (von 10⁷ Keimen auf nicht nachweisbar).

Die Textilien werden dabei in unterschiedlichen Verfahrensanordnungen dem Plasma ausgesetzt - es werden sowohl der Ionenwind als auch Ozon und Folgereaktionen, sowie kurzlebige Spezies genutzt.

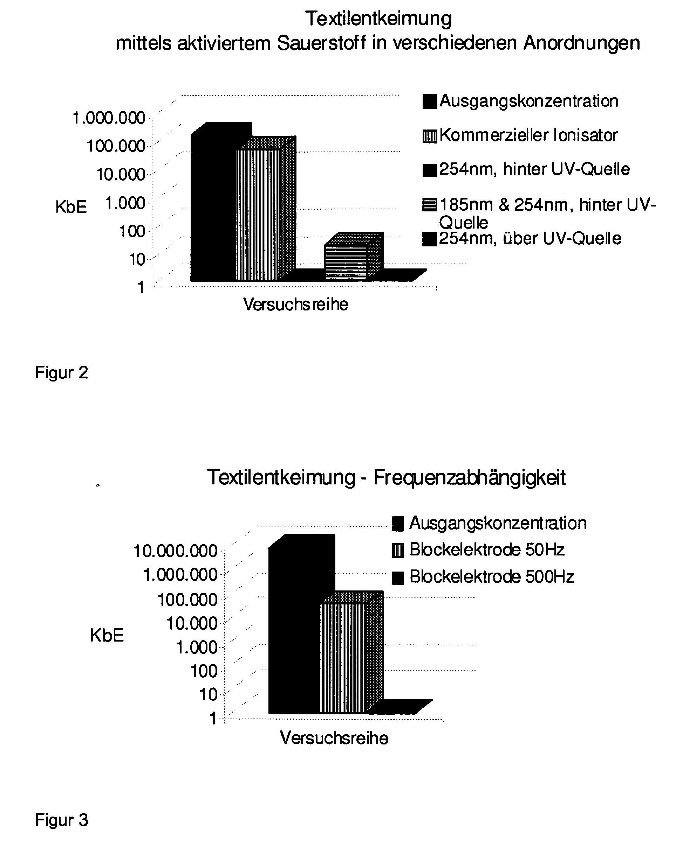

In Figur 2 wird Textilentkeimung mittels aktiviertem Sauerstoff in verschiedenen Anordnungen gezeigt. Die Dauer der Versuche betrug 2x15 min. Dabei wird aus dem Diagramm deutlich, dass bei Benutzung eines kommerziellen lonisators kaum Veränderungen registriert werden konnten. Es hat sich erwiesen, dass die Bestrahlung mit einer UV-Quelle mit einer Wellenlänge von 254nm die effektivsten Ergebnisse liefert, egal, ob diese Quelle vor oder unter dem zu behandelnden Gut liegt.

Figur 3 zeigt die Textilentkeimung mit Blockelektroden verschiedener Frequenzen. Auch hier betrug die Dauer der Versuche 2x15 min. Die Auswertung zeigt, dass bei 50 Hz nur eine geringe Entkeimung zu verzeichnen ist, aber bei einer Frequenz von 500 Hz so gut wie keine Keime mehr nachgewiesen werden können.

Oxidation und Zersetzung gasförmiger Komponenten

Im Falle gasförmiger Komponenten, die der Kleidung anhaften - wurde bei der Beseitigung von Küchengerüchen gezeigt, dass man unter eine belastende Geruchsschwelle gelangen kann. So wurden beispielsweise Aldehyde und MEK (Referenzsubstanz für Küchengerüche) abgebaut.

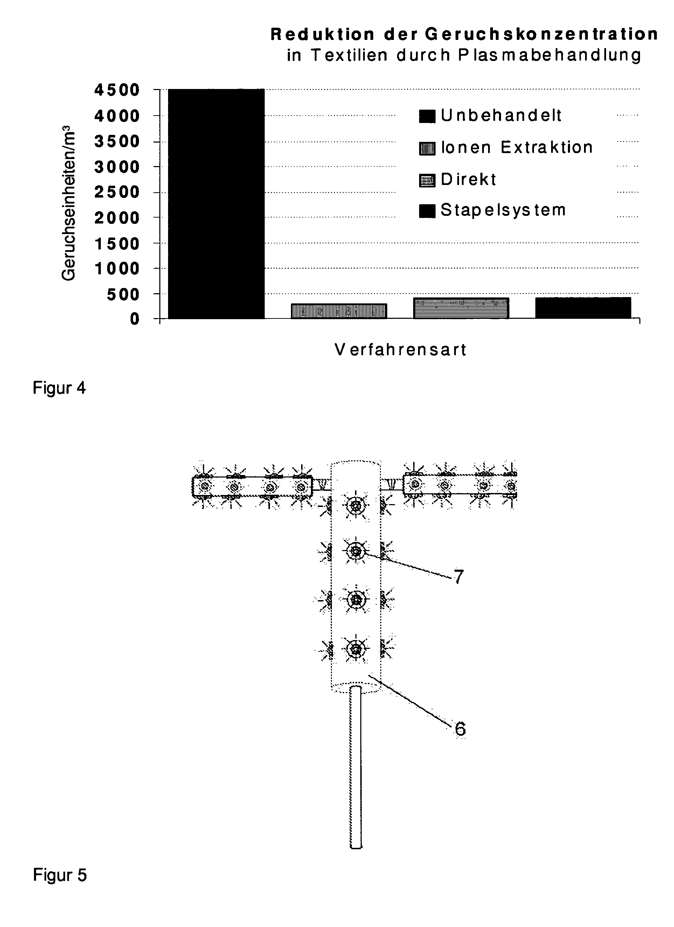

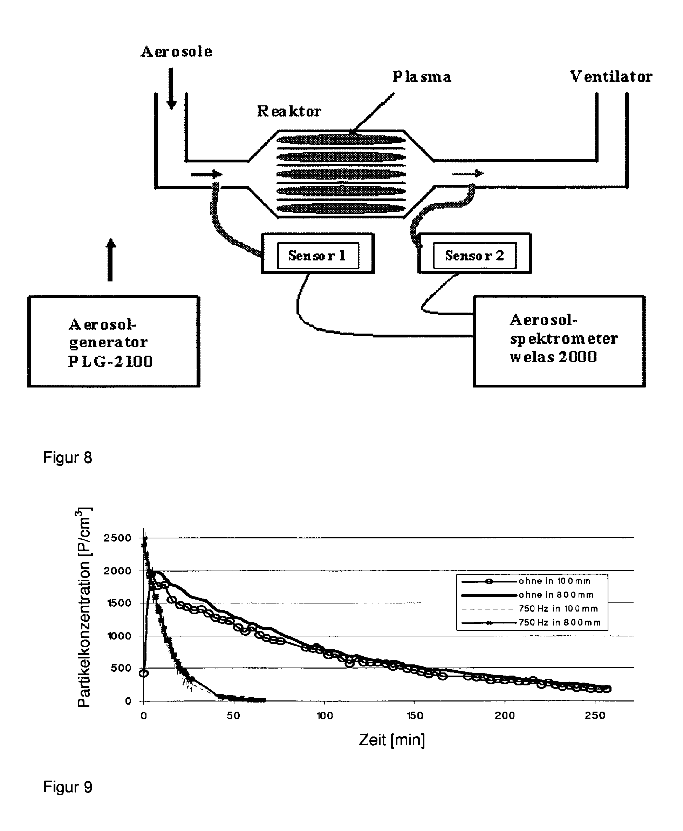

Figur 4 zeigt ein Diagramm, in dem die Reduktion der Geruchskonzentration in Textilien durch Plasmabehandlung dargestellt wird. Die Geruchseinheiten/m³ betragen unbehandelt 4500. Nach der Behandlung mit Ionen-Extraktion wurden die besten Ergebnisse gemessen. Aber auch mit einer direkten Plasmabehandlung und einer Behandlung im Stapelsystem, wie in Figur 8 gezeigt, wurden Werte unter 500 Geruchseinheiten/m³ gemessen.

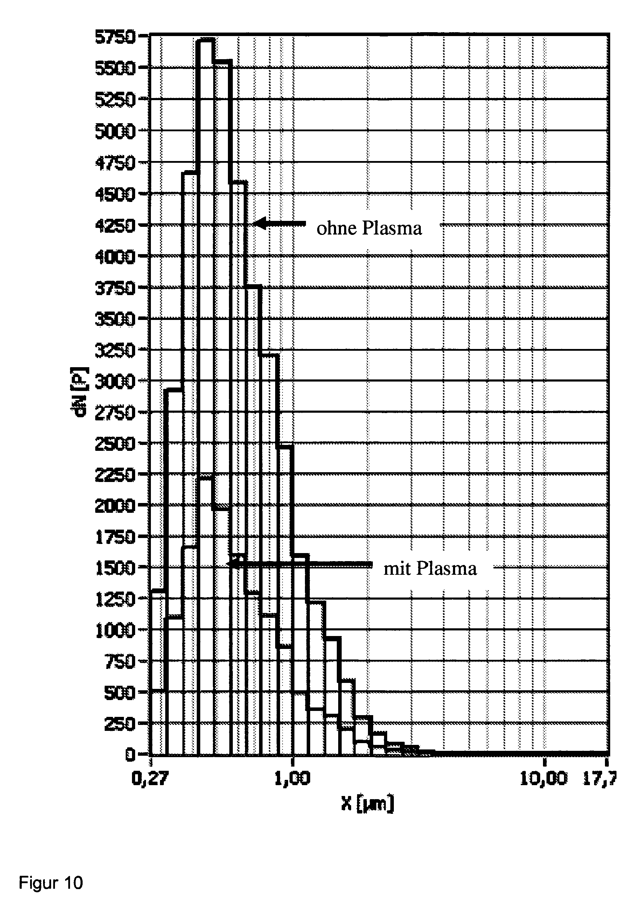

In Figur 10 wird hierzu ein Diagramm des Vergleichs ohne (blau) und mit (rot) Plasma im Stapelreaktor (Stapelsystem mit 50 Gittern) gezeigt.

Abscheidung von Keimen und Aerosolen und ihre Zersetzung

Die Aerosolabscheidung und Zersetzung ist ein weiteres wesentliches Merkmal des vorliegenden Verfahrens. Dies wird durch ein neuartiges Prinzip zur Ionen-Extraktion aus einem Plasma ermöglicht. Damit können Schmutzpartikel bis hin zu Bioaerosolen aufgeladen und nachfolgend in sehr kurzen Zeitbereichen abgeschieden bzw. wiederum einem anderen Plasma zur Zersetzung zugeführt werden.

Die aufgeführten Elemente erlauben in speziellen Anordnungen die Reinigung von geruchsbeladener Kleidung sowie die Entkeimung und das Bleichen von weißer Wäsche.

Das vorgeschlagene Prinzip eröffnet eine Vielzahl weiterer Möglichkeiten der Anwendung.

- Textilreinigung (z. Zt. Gerüche, leicht verschmutzt) im Heimbereich,
- Reinigung von Reinraumtextilien,
- Reinigung Medizintextilien,
- Reinigung von Schutzausrüstungen,
- Reinigung von Kleidung für Allergiker (Schleuse, Schrank).

Der Kundennutzen liegt insbesondere in der trockenen Textilreinigung. Es entfallen der aufwändige Wasch- und Trocknungsprozess und teilweise der Bügelvorgang.

In der Fig. 5 ist eine Vorrichtung dargestellt, mit der leicht verschmutzte oder von Gerüchen beladene Textilien gereinigt werden können. Die Vorrichtung besteht aus einem Körper 6 mit Düsen 7. Der Körper 6 ist hohl und geeignet für die Durchströmung von Luft, die durch die Düsen 7 dann austritt. Über diesen Körper können die zu reinigenden Kleidungsstücke gehängt werden. Im Inneren des Körpers ist ein Elektrodensystem zur Erzeugung eines Plasmas angebracht. Das Elektrodensystem ist bevorzugt eine Form von dielektrisch behinderter Entladung. Dort werden im Plasma reaktive Spezies wie Ozon erzeugt die dann duch die Düsen strömen. Zur Verstärkung der Wirksamkeit wird in den Düsen mit UV-Licht das Ozon photolytisch zersetzt und zu molekularem und atomarem Aktivsauerstoff umgewandelt, der besonders reaktiv ist.

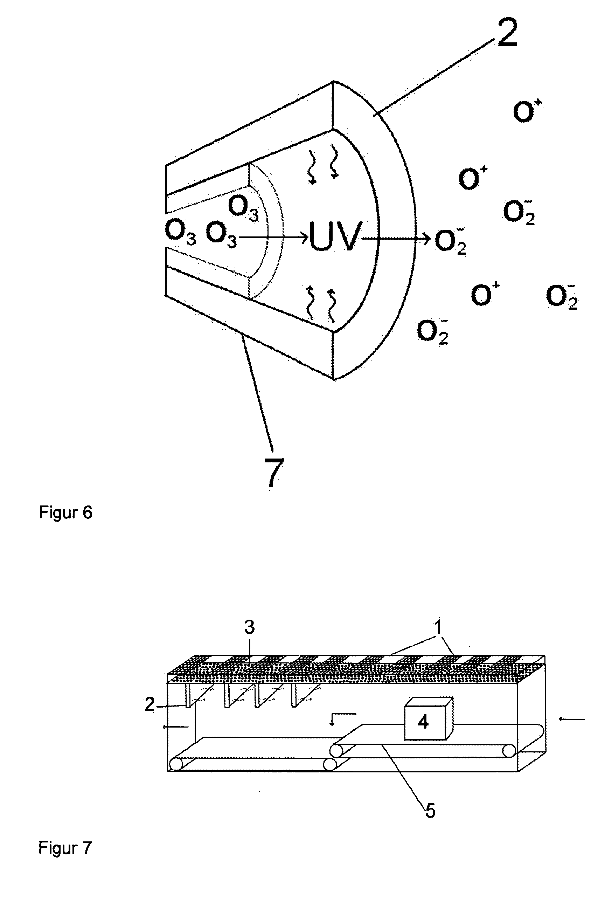

Die Fig. 6 zeigt schematisch die Funktionsweise der Düse. Durch die Anordnung der UV-Quelle in der Düse bzw. in ihrer Nähe ist gewährleistet, daß der Aktivsauerstoff lange genug lebt, um für Reinigungszwecke genutzt werden zu können.

Fig. 7 zeigt eine Anordnung, die als Schleuse ausgebildet ist. In der Decke der Schleuse sind Elektrodensysteme 1, bevorzugt Oberflächenentaldungskonfigurationen, bei denen durch Pulsspannungen Ionen aus dem Entladungsbereich extrahiert werden können. Für das Verfahren ist vorgesehen, daß die Polarität der Pulsspannung von Zeit zu Zeit wechselt, um eine einseitige Aufladung des in der Schleuse befindlichen Gutes zu vermeiden. Zur Extraktion von Ionen befindet sich unterhalb des Elektrodensystems ein Lochgitter 3. Das Lochgitter 3 kann auch andere Formen haben - wie beispielsweise gespannte Drähte oder Maschendraht. Auch als Hochspannungslinsen fungierende Blenden können eingesetzt werden.

Lochgitter 3 oder Drähte können in einer Ausführung auch von Isolationsmaterial, umgeben sein, um das unerwünschte abfließen von Ionen zu vermeiden.

Im Beispiel nach Fig. 7 ist ein Stückgut 4 dargestellt. Dies soll durch eine Fördereinrichtung 5 so bewegt werden, dass möglichst die komplette Oberfläche dem Plasma und den extrahierten Ionen ausgesetzt sind. Zur Verbesserung der Wirksamkeit ist eine UV-Lampe 2 vorgesehen. Hier ist insbesondere an eine flache Excimerlampe gedacht, die beispielsweise mit KrCl gefüllt ist und eine UV-Wellenlänge von 220 -230 nm abgibt oder eine UV-Lampe mit einer Füllung aus XeCl mit einem Wellenlängenbereich von 290 - 315 nm. Auch andere Lampen, die in diesem Wellenlängenbereich arbeiten können eingesetzt werden, wie beispielsweise Quecksilberlampen mit einer Wellenlänge von 254 nm.

Figur 9 zeigt den Abbau der Partikelkonzentration/m³ über die Zeit. Bei einer Frequenz von 750 Hz sind nach ca. 50 min keine Partikel mehr nachweisbar.

Die Anwendung ist nicht auf die Schleuse für Stückgüter beschränkt. So kann man verschiedene Textilien dort durchschicken - insbesondere Reinraumtextilien, Medizintextilien, Schutzkleidung oder auch Kleidung für Allergiker.

In einer speziellen Anordnung ist auch die direkte Begehung einer solchen Schleuse durch Menschen vorgesehen. Das ist insbesondere Allergiker vorteilhaft.

Die Plasmaschleuse ist in oder nach/vor weiträumigen Personenbeförderungssystemen wie Flugzeugen, Eisenbahn, Schiffen sowie Seuchengebieten einsetzbar.

Ferner ist das Prinzip einsetzbar in Gesundheitseinrichtungen - wie Reinluftdecken; sterile Kabinen (Zugang wieder mit Schleuse), Tierhaltung/Ställe. Weitere mögliche Gebiete: Lebensmittelindustrie, Halbleiterindustrie, Optikbereich; denkbar sind auch der Heimbereich (Allergien), Gewächshäuser und Lager für Obst, Gemüse sowie Getreide.

## Patentansprüche

1. Verfahren zur Textilreinigung und Desinfektion mittels Plasma mit den Schritten Ozonerzeugung und -aktivierung, Entkeimung, Oxidation und Zersetzung gasförmiger Komponenten, sowie Abscheidung und Zersetzung von Keimen und Aerosolen **dadurch gekennzeichnet, dass** eine Plasmagenerierung durch Pulsspannungen unterschiedlichen Vorzeichens erfolgt und dabei Ionen extrahiert werden.

2. Verfahren nach Anspruch 1 **dadurch gekennzeichnet, dass** abwechselnd positive oder negative Ionen extrahiert werden.

3. Verfahren nach einem der Ansprüche 1 bis 2 **dadurch gekennzeichnet, dass**
Keime und Aerosole durch die extrahierten Ionen elektrisch aufgeladen werden und so durch ein elektrisches Feld einem Abscheidebereich oder einer Plasmaeinheit zur Zersetzung zugeführt werden.

4. Verfahren nach einem der Ansprüche 1 bis 3 **dadurch gekennzeichnet, dass**
die Entkeimung durch ein oder mehrere der folgenden Komponenten wie das erzeugte Ozon, den Aktivsauerstoff, die extrahierten Ionen und UV-Strahlung erfolgt.

5. Verfahren nach einem der Ansprüche 1 bis 3 **dadurch gekennzeichnet, dass**
eine Oxidation und Zersetzung gasförmiger Komponenten durch aus dem Plasma extrahierte Ionen erfolgt.

6. Verfahren nach einem der Ansprüche 1 bis 5 **dadurch gekennzeichnet, dass**
eine zusätzliche Sauerstoffaktivierung durch eine UV-Lampe erfolgt.

7. Verfahren nach Anspruch 6 **dadurch gekennzeichnet, dass**
eine zusätzliche Sauerstoffaktivierung durch UV-Licht mit einer Wellenlänge im Bereich von 220 bis 230 nm erfolgt.

8. Verfahren nach Anspruch 6 **dadurch gekennzeichnet, dass** eine zusätzliche Sauerstoffaktivierung durch UV-Licht mit einer Wellenlänge im Bereich von 290 bis 315 nm erfolgt.

9. Verfahren nach Anspruch 6 **dadurch gekennzeichnet, dass** eine zusätzliche Sauerstoffaktivierung durch UV-Licht mit einer Wellenlänge von 254 nm erfolgt.

## Claims

1. Method for textile cleaning and disinfection by means of plasma including the steps of generation and activation of ozone, sterilisation, oxidation and decomposition of gaseous components as well as the deposition and decomposition of germs and aerosols, **characterized in that** a plasma generation takes place by means of pulse voltages of different signs, and as a result ions are extracted.

2. Method according to claim 1, **characterized in that**
positive and negative ions are extracted alternately.

3. Method according to any one of claims 1 to 2, **characterized in that**
germs and aerosols are electrically charged by said extracted ions, and thus are fed to a deposition area or a plasma unit for decomposition by an electric field.

4. Method according to any one of claims 1 to 3, **characterized in that**
said sterilisation takes place by one or more of the following components such as said generated ozone, active oxygen, extracted ions, and ultraviolet radiation.

5. Method according to any one of claims 1 to 3, **characterized in that**
said oxidation and decomposition of gaseous components takes place by means of ions extracted from said plasma.

6. Method according to any one of claims 1 to 5, **characterized in that**
an additional activation of oxygen takes place by a ultraviolet light source.

7. Method according to claim 6, **characterized in that**
an additional activation of oxygen by means of ultraviolet light having a wave length in the range of 220 to 230 nm takes place.

8. Method according to claim 6, **characterized in that**
an additional activation of oxygen by means of ultraviolet light having a wave length in the range of 290 to 315 nm takes place.

9. Method according to claim 6, **characterized in that**
an additional activation of oxygen by means of ultraviolet light having a wave length of 254 nm takes place.

## Revendications

1. Procédé de nettoyage et de désinfection de textiles par plasma, comportant les étapes de production et d'activation d'ozone, de stérilisation, d'oxydation et de décomposition de composantes gazeuses, ainsi que de précipitation et de décomposition de germes et d'aérosols, **caractérisé en ce qu'**une génération de plasma est effectuée par des tensions d'impulsions de différents signes et des ions sont alors extraits.

2. Procédé selon la revendication 1, **caractérisé en ce que** des ions positifs ou des ions négatifs sont extraits en alternance.

3. Procédé selon l'une des revendications 1 à 2, **caractérisé en ce que** des germes et des aérosols sont électriquement chargés par les ions extraits et amenés ainsi par un champ électrique à une zone de précipitation ou à une unité de plasma pour une décomposition.

4. Procédé selon l'une des revendications 1 à 3, **caractérisé en ce que** la stérilisation est effectuée par une ou plusieurs des composantes suivantes telles que l'ozone produit, l'oxygène actif, les ions extraits et le rayonnement ultraviolet.

5. Procédé selon l'une des revendications 1 à 3, **caractérisé en ce qu'**une oxydation et une décomposition de composantes gazeuses sont effectuées par des ions extraits hors du plasma.

6. Procédé selon l'une des revendications 1 à 5, **caractérisé en ce qu'**une activation d'oxygène additionnelle a lieu par une lampe à ultraviolet.

7. Procédé selon la revendication 6, **caractérisé en ce qu'**une activation d'oxygène supplémentaire est effectuée par de la lumière ultraviolette avec une longueur d'onde dans la plage de 220 à 230 nm.

8. Procédé selon la revendication 6, **caractérisé en ce qu'**une activation d'oxygène supplémentaire est effectuée par de la lumière ultraviolette avec une longueur d'onde dans la plage de 290 à 315 nm.

9. Procédé selon la revendication 6, **caractérisé en ce qu'**une activation d'oxygène supplémentaire est effectuée par de la lumière ultraviolette avec une longueur d'onde de 254 nm.
